Europäisches Patentamt

European Patent Office

Office européen des brevets

(11) Publication number: **0 397 485**
**A1**

# EUROPEAN PATENT APPLICATION

(21) Application number: **90305025.0**

(22) Date of filing: **10.05.90**

(51) Int. Cl.⁵: **C12N 15/85, C12N 15/86, C12P 21/00**

(30) Priority: **12.05.89 GB 8910962**

(43) Date of publication of application:
**14.11.90 Bulletin 90/46**

(84) Designated Contracting States:
**AT BE CH DE DK ES FR GB GR IT LI LU NL SE**

(71) Applicant: **NATURAL ENVIRONMENT RESEARCH COUNCIL**
**Polaris House North Star Avenue**
**Swindon SN2 1EU(GB)**

(72) Inventor: **Possee, Robert David, Dr.**
**8 Hurdeswell**
**Long Hanborough, Oxon(GB)**
Inventor: **Hill-Perkins, Michele Susanne, Dr.**
**9 Wyndham Way**
**Oxford OX2 8DF(GB)**

(74) Representative: **Ritter, Stephen David et al**
**Mathys & Squire 10 Fleet Street**
**London EC4Y 1AY(GB)**

(54) **Novel baculovirus expression vectors and use thereof in the expression of foreign proteins in insects or insect cells.**

(57) The invention providres a recombinant expression vector for expressing a foreign protein in insects or insect cells comprising DNA sequences coding for the foreign protein operatively linked under the transcriptional control a promoter which is active at a stage of the infection of the insects or insect cells which precedes the stage at which the polyhedrin and p10 promoters are active.

EP 0 397 485 A1

# NOVEL BACULOVIRUS EXPRESSION VECTORS AND USE THEREOF IN THE EXPRESSION OF FOREIGN PROTEINS IN INSECTS OR INSECT CELLS

This invention relates to novel baculovirus expression vectors and use thereof in the expression of foreign proteins in insects or insect cells.

Baculoviruses have been isolated from many invertebrates including Lepidoptera, Hymenoptera, Diptera, Coleoptera and Crustacea. Particular attention has been paid to those viruses from insects, principally with a view to their use as control agents of insect pests in agriculture and in the development of expression systems for expression of foreign proteins. The viruses have a complex structure consisting of a genome of covalently closed circular DNA of about 128 kilobase pairs (kbp) packaged in a rod-shaped nucleocapsid. The nucleocapsids are enclosed within a lipoprotein envelope to form virus particles which, in turn, are further packaged into large proteinaceous inclusion bodies called polyhedra (Kelly, 1985). These structures are very stable and allow the virus to persist in the environment for many years before encountering a susceptible host.

A baculovirus vector system based on the nuclear polyhedrosis virus of *Autographa californica* (AcNPV) which utilises the promoter from the baculovirus polyhedrin gene to drive expression of foreign genes in infected cells is described in US-A-4,745,015 (see also Luckow and Summers, 1988). Further developments of the baculovirus vector system have made use of the promoter from the AcNPV p10 gene to produce β-galactosidase protein (Vlak *et al.*, 1988). With both promoters, the strategy for developing the expression vectors has involved deleting at least part of the coding sequences of polyhedrin or p10 genes and replacing them with the foreign gene of choice. A consequence of this is that respectively the final recombinant baculoviruses generally lack the polyhedrin or p10 gene products. Fortuitously, both are dispensible for virus replication and growth of the recombinants is not affected (Smith *et al.*, 1983; Vlak *et al.*, 1988).

Although the vectors based on the polyhedrin and p10 promoters appear capable of correctly processing foreign genes in the infected cell there is evidence that in the case of glycosylation this process does not entirely mimic the normal mammalian cell. In the case of the influenza virus haemagglutinin the protein produced in insect cells is of a slightly lower apparent molecular weight than the same product in mammalian cells (Kuroda *et al.*, 1986; Possee, 1986). Kuroda *et al.* (1986) concluded that the oligosaccharide side chains added to the insect derived HA were not identical to those attached in the vertebrate cells.

A disadvantage of the use of expression systems based on the polyhedrin and p10 promoters is that these promoters are so-called "very late" promoters which become active at a terminal phase of the infection and just before cell death, thus precluding an extended time of expression of the desired protein.

We have now developed a novel expression vector which overcomes this disadvantage.

According to one aspect of the present invention there is provided a recombinant expression vector for expressing a foreign protein in insects or insect cells comprising DNA sequences coding for the foreign protein operatively linked under the transcriptional control of a promoter which is active at a stage of the infection of the insects or insect cells which precedes the stage at which the polyhedrin and p10 promoters are active.

The promoter of the expression vectors of the invention may be a naturally occurring promoter or a synthetic promoter. By the term "naturally occurring promoter" is meant a DNA sequence which is identical to the sequence of a promoter region of the genome of a naturally occurring baculovirus. By "synthetic promoter" is meant a DNA sequence which is not identical to the sequence of a promoter region of the genome of a naturally occurring baculovirus, but nonetheless is structurally related to, or is derived from, or is designed to mimic such a sequence and thereby is active as a promoter. Preferably the DNA sequence of such a synthetic promoter has structural and functional similarity to the sequence of a promoter region of the genome of a naturally occurring virus. Differences in sequence of a synthetic promoter by comparison with a natural promoter may consist of base deletions, insertions, duplications, or substitutions and may result from intentional mimicing or "tailoring" natural sequences or be derived by mutation or other standard procedures of genetic manipulation.

Naturally occurring baculovirus promoters fall into four categories according to the stage at which they become active in the infection cycle. These have been termed the immediate early, delayed early, late and very late promoters. The polyhedrin and p10 promoters fall into the "very late" category. Preferably the promoter of the expression vector of the invention falls into the "late" category. Most preferably the promoter of the expression vector of the invention is the promoter of the baculovirus basic protein or a synthetic promoter structurally related to the said promoter.

The basic protein is an arginine-rich structural core protein found closely associated with the viral DNA (Tweeten et al., 1980; Bud and Kelly, 1980). For AcNPV the protein has an apparent molecular weight of about 12.5 kilaDalton (KD) and is rich in the basic amino acid arginine. Hence it is called the basic protein. Evidence that it is virus encoded has been provided by Wilson et al. (1987) who identified an open reading frame encoding an arginine-rich 6.9 KD protein in the Hind III-H/Eco RI-D region of the AcNPV genome. The discrepancy in the calculated (6.9 KD) and observed (12.5 KD) molecular weights was concluded to be due to the aberrant migration of the highly basic protein in polyacrylamide gels. Furthermore, a synthetic peptide to the predicted 6.9 KD translation product induced antibodies in rabbits which reacted with the basic protein from purified virus particles in Western blot analyses (Wilson, 1988). It has been reported that the basic protein is first detected at 6-12 h.p.i. (hours post infection) but is synthesized at its maximum rate between 12-32 h.p.i. and is one of the seven most abundant virus induced proteins in infected cells (Rohel and Faulkner, 1984).

The invention will now be described with particular reference to the accompanying drawings of which:

Figure 1 illustrates the construction of expression vectors according to the invention

Figure 2 shows the nucleotide sequences of a portion of the genome of Autographa californica nuclear polyhedrosis virus, including squences of the basic protein promoter, and

Figure 3 shows photographs of autoradiograms of polyacrylamide gels illustrating the use of the basic protein promoter to control expression of a foreign protein (the Escherichia coli β-galactosidase) by comparison with the expression of AcNPV-induced proteins.

Figure 4 shows a photograph of stained polyacrylamide gels illustrating the accumulation of a foreign gene product (E. coli β-galactosidase) expressed from the basic protein promoter by comparison with the same gene expressed from the polyhedrin promoter.

In carrying out the invention a transfer vector was first constructed utilising a pAcRP transfer vector (Possee, 1986; Possee and Howard, 1987). The construct included the basic protein promoter region in place of the polyhedrin promoter. As an example of the ability of this copy of the basic protein promoter to express a foreign gene the E. coli β-galactosidase gene was inserted downstream of the basic protein promoter in the transfer vector and then transferred to the genome of AcNPV to make a recombinant baculovirus. Infection of insect cells with the recombinant baculovirus yields high level expression of the β-galactosidase protein. The expression followed the same kinetics as those of the native basic protein. The protocol is described in more detail in the following example.

## A. Sequence of the basic protein gene and its promoter

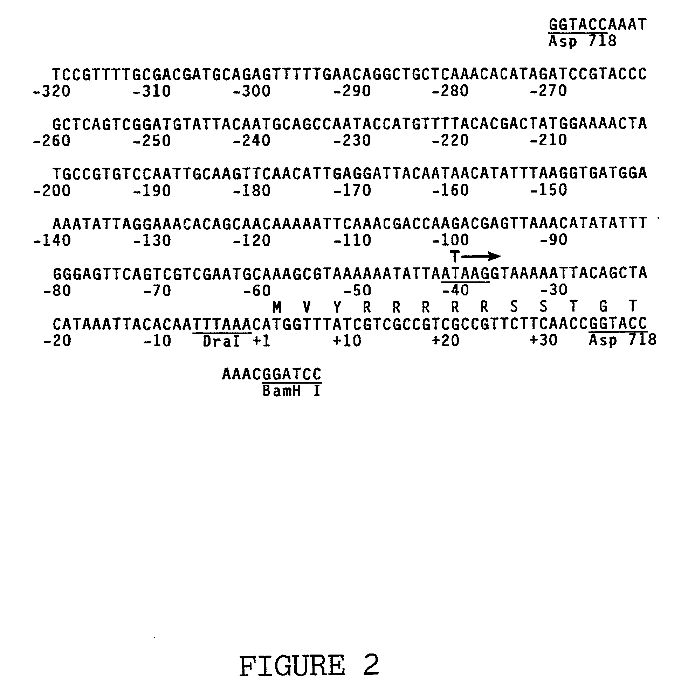

The AcNPV arginine-rich polypeptide, or basic protein has been mapped to the Hind III-H fragment in the virus genome and sequenced (Wilson et al., 1987; Possee, unpublished data). In the report from Wilson et al. (1987) the protein coding and putative promoter sequences from AcNPV strain L1 span an Asp 718 (Kpn I) site within the Hind III-H region. By comparison with the L1 strain the C6 strain of AcNPV used in this study has an additional Asp 718 site in the virus DNA that is some 364 nucleotides further upstream to the coding region of the basic protein. We determined from sequence analysis of this 364 nucleotide fragment that it contained the putative basic protein promoter, transcription initiation site and the 5′ end of the coding region (Fig. 2).

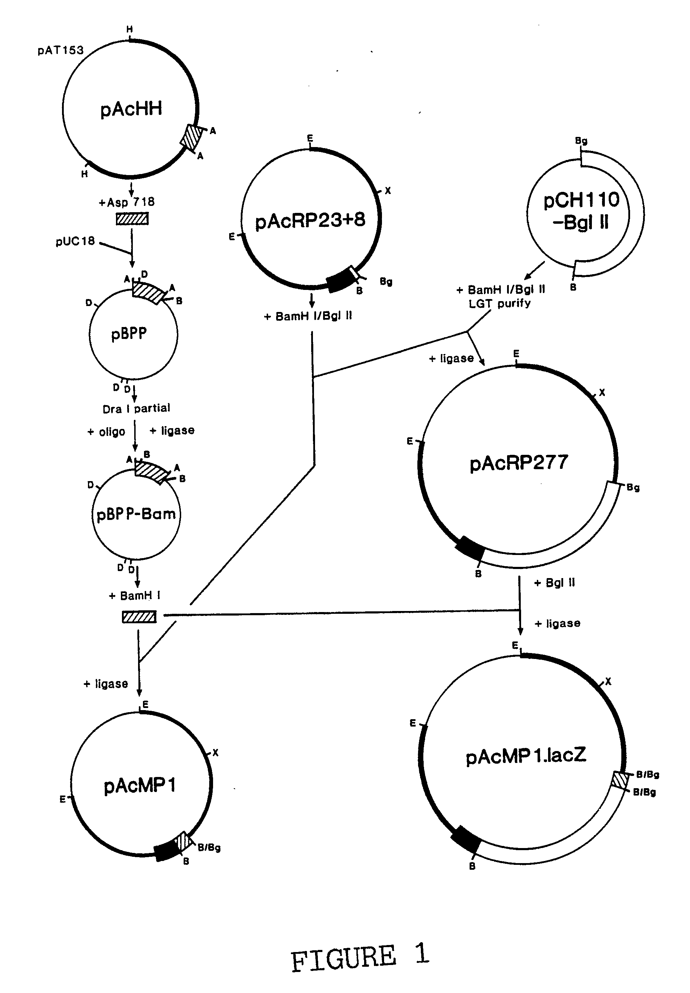

Specifically, the basic protein promoter region of AcNPV was sequenced as follows: Referring to Figure 1, the Hind III-H fragment of AcNPV DNA was inserted into pAT153 using standard procedures (Maniatis et al., 1982). A CsCl gradient-purified stock of the recombinant plasmid was digested with Asp 718 (an isoschizomer of Kpn I) to produce 3 fragments. The smallest of these (364 nucleotides) was sequenced and shown to contain the 5′ coding and upstream region of the basic protein gene (Fig. 1). This fragment was inserted into pUC18 at the Asp 718 site, amplified and purified on a caesium chloride gradient. This plasmid was designated pBPP.

## B. Construction of recombinant transfer vectors

It was considered unlikely that replacing the coding region of the basic protein gene of AcNPV with a foreign gene would result in the production of a viable virus recombinant since the basic protein gene is considered to be an essential gene in view of the fact that the basic protein is intimately associated with the packaging of virus DNA (Tweeten et al., 1980; Bud and Kelly, 1980). Therefore it was decided to duplicate the basic protein promoter in an alternative position within the virus genome, a position selected so as not to affect virus replication. The site chosen was the position of the polyhedrin gene, where deletion of the polyhedrin coding sequences (Smith et al., 1983) or deletion of its promoter elements (Possee and Howard, 1987) does not affect the replication of the virus.

A BamH I restriction site was created at the end of the 5' leader sequence of the basic protein gene by annealing two synthetic complementary oligonucleotides (5' AAACGGATCC and 3' TTTGCCTAGG) and blunt-end ligating them on the three T residues of the Dra I site located immediately upstream of the ATG initiation codon (Fig. 1). The products were used to transform JM105 cells thus deriving the plasmid pBPP-Bam (Fig. 1). The two oligonucleotides were designed so that insertion in one orientation would recreate the original Dra I site and 5' leader sequence (to the -1 nucleotide, counting the ATG as + 1, + 2, + 3) and add a BamH I site for insertion of a foreign gene. Previous studies (Matsuura et al., 1987) on the function of the 5' upstream region of the polyhedrin gene of AcNPV have shown that high levels of protein expression are obtained by recombinants possessing the complete 5' leader sequence; therefore it was considered prudent to preserve the sequence of the analogous region in the basic protein. Specifically, to prepare the pBPP-Bam vector, and since the pBPP plasmid has four Dra I sites, the construct pBPP was incubated with Dra I under conditions which produced incomplete digestion of the plasmid (0.1-0.5 units of Dra I per μg pBB DNA incubated at 37°C for 1 hr). This resulted in a mixture of linear full-length and smaller products; the full-length linear species having been digested at any one of the four Dra I sites. The full-length species were resolved in a low gelling temperature agarose gel (LGT) and purified by phenol extraction from the melted agarose (Possee and Kelly, 1988). The linear molecules were then treated with calf intestinal phosphatase (Maniatis et al., 1982) which was removed by phenol extraction after the incubation. The DNA preparation was then annealed with the complementary oligonucleotides described above and the products used to transform JM105 cells. Following transformation individual bacterial colonies containing recombinant plasmids were amplified in liquid medium and the cloned DNA purified for analysis with restriction enzymes to determine whether the synthetic oligonucleotide was inserted at the single Dra I site within the basic protein promoter, or at one of the three other sites in pBB. Insertion of the oligonucleotides at the Dra I site immediately preceeding the coding region of the basic protein gene (Fig. 1) in recombinant plasmid designated pBB-Bam restored the Dra I site and created a Bam HI site at the normal position of the ATG translation initiation codon.

A fragment of 328 base-pairs was then excised by digestion of pBPP-Bam with the enzyme Bam HI and purified from an LGT gel. This DNA contained the putative basic protein promoter and a small region of the pUC18 polylinker. The fragment was inserted into the Bgl II site of vector pAcRP277 to produce the recombinant transfer vector pAcMP1.lacZ. pAcRP277 was previously derived from pAcRP23.lacZ (Possee and Howard, 1987) and consists of the EcoR I-I fragment from AcNPV DNA with the complete polyhedrin promoter and the first 170 base pairs of the coding squences removed and replaced with the coding sequences of the E. coli β-galactosidase (lacZ) gene. By cutting the pAcRP277 with Bgl II and inserting the Bam HI derived 328 base-pair fragment the Bgl II site was destroyed (Fig. 1: B/Bg fused sequences). The recombinant pAcMP1.lacZ thus contained the basic protein promoter in front of the β-galactosidase gene.

The second, general purpose transfer vector, pAcMP1, for expressing any foreign gene under the control of the basic protein promoter was derived by inserting the 328 base-pair basic protein promoter fragment described above into a modification of pAcRP23 (the so-called pAcRP23 + 8, Fig. 1). This vector was derived from pAcRP23 by the insertion of a Bgl II linker (described in Possee and Howard, 1987). Digestion of pAcRP23 + 8 with Bam HI and Bgl II and insertion of the 328 base-pair fragment destroyed the Bgl II site (upstream of the inserted sequence) and formed the vector pAcMP1 creating a unique Bam HI site for inserting a foreign gene under the control of the basic protein promoter.

C. Preparation and selection of recombinant viruses expressing the lac Z gene

The transfer vector pAcMP1.lacZ, containing the β-galactosidase gene under the control of the basic protein promoter, was co-transfected with infectious AcNPV DNA according to previously described protocols (Possee, 1986). The transfected cells were harvested after two days and the progeny virus titrated in S. frugiperda cells. Plaques containing recombinant viruses expressing β-galactosidase were identified by incubating the plates with the enzyme substrate X-gal. Blue plaques which also lacked polyhedra were isolated and further purified by three rounds of titration in S. frugiperda cells. Large stocks of the genetically homogenous recombinant viruses were obtained by propagation in spinner cultures of S. frugiperda cells.

D. Analysis of proteins from infected S. frugiperda cells

Spodoptera frugiperda cells ($10^6$ per 35 mm diameter culture dish) were inoculated with virus at a multiplicity of infection of 10 pfu per cell, or with medium for a mock infected control. After 1 hr at room temperature (21°C) the virus was removed and replaced with 2 ml TC100/10% FCS and the cells incubated at 28°C. At the appropriate times after infection the cells were pulse labelled for 1 hr with [$^{14}$C]arginine, the cells displaced from the dish into the medium, pelleted by low speed centrifugation and washed once with cold PBS. The cells were resuspended in 100 $\mu$l TE and lysed by boiling in dissociation buffer (30 mM Tris-HCl, pH6.9, 5% 2-mercaptoethanol, 5% SDS, 5% glycerol, 0.01% bromophenol blue) for 5 minutes. Samples were analysed in 10-30% polyacrylamide gradient gels (Cook et al., 1979). Protein gels were stained with 0.2% Coomassie brilliant blue (Biorad) in 10% glacial acetic acid, 45% methanol and 45% water.

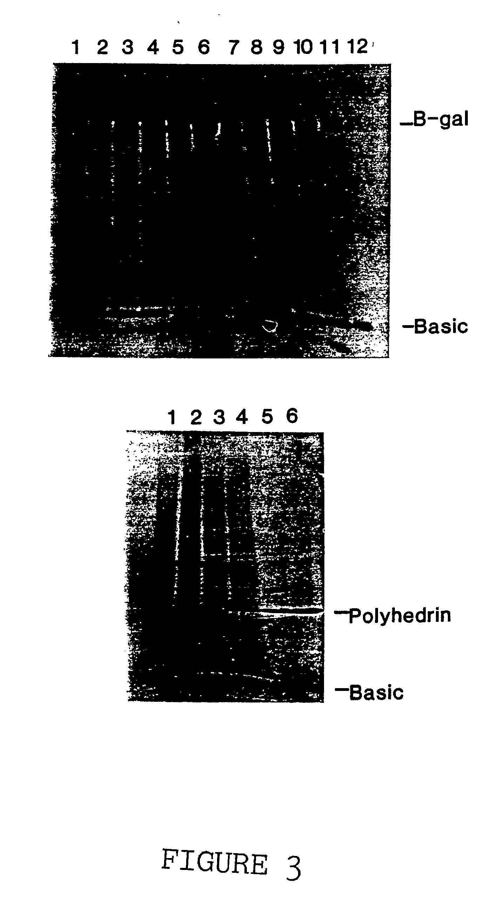

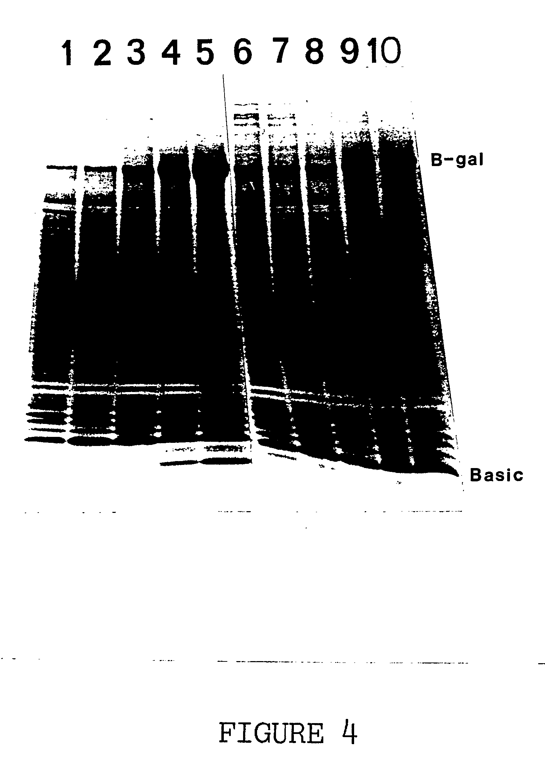

The efficiency of the basic protein promoter in directing foreign gene expression by comparison with the same gene expression obtained using the polyhedrin promoter was assessed by infecting cells with AcMP1.lacZ orAcRP23.lacZ. The results were compared to the protein data obtained with AcNPV. Proteins were analysed at 8, 12, 15, 18, 24 and 30 hours after infection. Figure 3 shows autoradiographs of polyacrylamide gels of the [$^{14}$C]-arginine-labelled proteins. Fig. 4 shows gels of AcRP23.lacZ or AcMP1.lacZ infected cell extracts stained with Coomassie blue. The level of $\beta$-galactosidase protein produced by the basic protein promoter (Fig. 3, top panel, AcMP1.lacZ, lanes 8,9) is higher between 12 and 15 hours post-infection than that produced by the polyhedrin promoter based vector (Fig. 3, top panel, AcRP23.lacZ, lanes 2, 3), thereafter the amount present in cells infected with AcMP1.lacZ increased slightly by 24 h.p.i. but remained constant thereafter. The AcRP23.lacZ virus continued to accumulate $\beta$-galactosidase protein until at least 30 h.p.i. (Fig. 3,4). The course of synthesis of $\beta$-galactosidase by AcMP1.lacZ is paralleled by the production of the basic protein. Synthesis of basic protein in all three virus infected cells is similar.

The above data demonstrate that a baculovirus expression vector derived from the AcNPV basic protein promoter has been produced and used to synthesize $\beta$-galactosidase protein in S. frugiperda cells. The advantage of this particular vector is that foreign proteins may be expressed in infected cells at earlier times after infection than is possible with the standard baculovirus vectors based on the polyhedrin or p10 promoters. The amount of foreign protein produced is comparable to that of the normal basic protein gene product; furthermore, the kinetics of synthesis are very similar. This vector may also be advantageous when producing proteins requiring extensive post translational modification (e.g., glycosylation and proteolytic cleavage) since the cell may be better equipped to cope with such processes earlier in infection rather than late when it is committed to the synthesis of polyhedrin and p10 proteins; neither of which apparently require extensive post-translational modification. The basic protein expression system according to the invention may also be advantageously used for designing genetically engineered virus insecticides. It will be feasible to express insect-specific toxins and hormones in the infected insect at earlier times than would be possible with the polyhedrin expression system, thus ensuring the faster possible effect on the target insect. The vector will also be useful in the design of multiple expression systems using both basic, polyhedrin and p10 promoters. It will be possible to express genes in an ordered sequence and thus permit the prior synthesis of proteins which can then play a role in processing those produced later in the infection cycle.

Plasmid pAcMP1 is deposited at the National Collection of Industrial and Marine Bacteria under Deposit No NCIMB 40185.

REFERENCES

BUD, H.M. & KELLY, D.C. (1980). An electron microscope study of partially lysed baculovirus nucleocapsids: The intranucleocapsid packaging of viral DNA. *Journal of Ultrastructure Research* 73,361-368.

COOK, R.F., AVERY, R.J. & DIMMOCK, N.J (1979). Infection of chicken erythrocytes with influenza and other viruses.*Infection and Immunity* 25, 396-402.

KELLY, D.C. (1985). The structure of physical characteristics of baculoviruses. In: *Viral Insecticides for Biological Control*, pp. 469-488. Edited by K.E. Sherman & K. Maramorosch. New York & London: Academic Press.

KURODA, K., HAUSER, C., ROTT, R., KLENK, H.-D. & DOERFLER, W. (1986). Expression of the influenza virus haemagglutinin in insect cells by a baculovirus vector. *European Molecular Biology Organisation Journal* 5, 1359-1365.

LUCKOW, V.A. & SUMMERS, M.D. (1988). Trends in the development of baculovirus expression vectors. *Bio/Technology* 6,47-55.

MANIATIS, T., FRITSCH, E.F. & SAMBROOK, J. (1982). *Molecular Cloning: A Laboratory Manual*. Cold Spring Harbor Laboratory. New York: Cold Spring Harbor Laboratory.

MATSUURA, Y., POSSEE, R.D., OVERTON, H.A. & BISHOP, D.H.L. (1987). Baculovirus expression vectors: the requirements for high level expression of proteins, including glycoproteins. Journal of General Virology 67, 1233-1250.

POSSEE, R.D. (1986). Cell-surface expression of influenza virus haemagglutinin in insect cells using a baculovirus vector. *Virus Research* 5,43-59.

POSSEE, R.D. & HOWARD, S.C. (1987). Analysis of the polyhedrin gene promoter of the *Autographa californica* nuclear polyhedrosis virus. *Nucleic Acids Research* 15,10233-10248.

POSSEE, R.D. & KELLY, D.C. (1988). Physical maps and comparative DNA hybridization of *Mamestra brassicae and Panolis flammea* nuclear polyhedrosis virus genomes. *Journal of General Virology* 69,1285-1298.

ROHEL, D.Z. & FAULKNER, P. (1984). Time course analysis and mapping of *Autographa californica* nuclear polyhedrosis virus transcripts. *Journal of Virology* 50,739-747.

SMITH, G.E., FRASER, M.J. & SUMMERS, M.D. (1983). Molecular engineering of the *Autographa californica* nuclear polyhedrosis virus genome: deletion mutations within the polyhedrin gene. *Journal of Virology* 46,584-593.

TWEETEN, K.A., BULLA, L.A. Jr. & CONSIGLI, R.A. (1980). Characterization of an extremely basic protein derived from granulosis virus nucleocapsids. *Journal of Virology* 33,866-876.

VLAK, J.M., KLINKENBERG, F.A., ZAAL, K.J.M., USMANY, M., KLINGE-ROODE, E.C., GEERVLIET, J.B.F., ROOSIEN, J. & VAN LENT, J.W.M. (1988). Functional studies on the p10-B-galactosidase fusion gene. *Journal of General Virology* 69,765-776.

WILSON, M.E., MAINPRIZE, T.H., FRIESEN, P.D. & MILLER, L.K. (1987). Location, transcription and sequence of a baculovirus gene encoding a small arginine-rich polypeptide. *Journal of Virology* 61,661-666.

WILSON, M.E. (1988). A synthetic peptide to the predicted 6.9K translation product of the Hind III-H/EcoR I-D region of the AcNPV genome induces antibodies to the basic DNA-binding protein. *Virus Research* 9,21-31.

FIGURE LEGENDS

Figure 1. Construction of pAcMP1.lacZ and the transfer vector pAcMP1. A schematic representation of the procedures used to derive pAcMP1.lacZ and pAcMP1 is shown (see text). The basic protein promoter fragment is depicted as a hatched box throughout the various stages, the lacZ coding region by an open box, and the carboxy half of the (out-of-frame) polyhedrin gene as a solid box.

Figure 2. Nucleotide sequence of the basic protein promoter. The sequence of the 364 base pair fragment produced by Asp 718 digestion of pAcHH (Fig. 1) is shown. It contains the transcription initiation start site (T arrow) and the amino terminal end of the coding sequences of the basic protein gene with the initial amino acid residues (MVYR. etc.) indicated above the DNA sequence beginning at nucleotide + 1. The Dra I site used to insert the synthetic oligonucleotide is indicated, together with the sequence of the oligonucleotide used to insert a Bam HI site after the Dra I site (bottom line).

Figure 3. (a) Expression of $\beta$-galactosidase by recombinant viruses. *Spodoptera frugiperda* cells were inoculated with 10 pfu/cell of AcRP23.lacZ (top panel, lanes 1-6) or AcMP1.lacZ (lanes 7-12). After adsorbtion for one hour at room temperature the cells were incubated at 28° C until 8, 12, 15, 18, 24 and 30 h.p.i. (lanes 1-6, AcRP23.lacZ; lanes 7-12, AcMP1.lacZ). Infected cells were pulse-labelled with [$^{14}$C]arginine and analysed in a 10-30% polyacrylamide gradient gel. B-gal: $\beta$-galactosidase; Basic: basic protein.
(b) Expression of polyhedrin protein by AcNPV. Cells were infected with AcNPV and pulse-labelled as described above at 8, 12, 15, 18, 24 and 30 h.p.i. (lanes 1 to 6).

Figure 4. Total protein expressed by recombinant viruses. Cells were infected with AcRP23.lacZ (lanes 1-5) or AcMP1.lacZ (lanes 6-10) as described in Fig. 3 and harvested at 12, 15, 18, 24 and 30 h.p.i. for analysis of intracellular proteins using Coomassie blue stained polyacrylamide gradient gels. B-gal: $\beta$-galactosidase; Basic: basic protein.

## Claims

1. A recombinant expression vector for expressing a foreign protein in insects or insect cells comprising DNA sequences coding for the foreign protein operatively linked under the transcriptional control of a promoter which is active at a stage of the infection of the insects or insect cells which precedes the stage at which the polyhedrin and p10 promoters are active.

2. A recombinant expression vector according to Claim 1 wherein the promoter is a naturally occurring promoter.

3. A recombinant expression vector according to Claim 2 wherein the naturally occurring promoter comprises a DNA sequence which is identical to the sequence of a promoter region of the genome of a naturally occurring virus.

4. A recombinant expression vector according to any preceding claim wherein the naturally occurring promoter is the basic protein promoter of *Autographa californica* nuclear polyhedrosis virus.

5. A recombinant expression vector according

**EP 0 397 485 A1**

to Claim 1 wherein the promoter is a synthetic promoter.

6. A recombinant expression vector according to Claim 4 wherein the promoter comprises a DNA sequence which is not identical to the sequence of a promoter region of the genome of a naturally occurring virus, but is structurally related to such a sequence and is active as a pomoter.

7. A recombinant expression vector according to Claim 5 wherein the promoter comprises a DNA sequence which comprises at least 75% of the natural sequence.

8. A recombinant expression vector according to Claim 6 wherein the promoter comprises a DNA sequence which comprises at least 75% of the natural sequence.

9. A recombinant expression vector according to any of Claims 2 to 8 wherein the naturally occurring promoter is the basic protein promoter of *Autographa californica* nuclear polyhedrosis virus.

10. A recombinant expression vector according to any of Claims 2 to 9, wherein the naturally occurring promoter includes sequences as set forth in Fig. 2.

11. A process for producing protein which comprises infecting insects or insect cells with a recombinant expression vector as claimed in any preceding claim.

FIGURE 1

EP 0 397 485 A1

```
                                                              GGTACCAAAT
                                                              Asp 718

    TCCGTTTTGCGACGATGCAGAGTTTTTGAACAGGCTGCTCAAACACATAGATCCGTACCC
-320      -310       -300       -290       -280       -270

    GCTCAGTCGGATGTATTACAATGCAGCCAATACCATGTTTTACACGACTATGGAAAACTA
-260      -250       -240       -230       -220       -210

    TGCCGTGTCCAATTGCAAGTTCAACATTGAGGATTACAATAACATATTTAAGGTGATGGA
-200      -190       -180       -170       -160       -150

    AAATATTAGGAAACACAGCAACAAAAATTCAAACGACCAAGACGAGTTAAACATATATTT
-140      -130       -120       -110       -100       -90
                                                   T——▶
    GGGAGTTCAGTCGTCGAATGCAAAGCGTAAAAAATATTAATAAGGTAAAAATTACAGCTA
-80       -70        -60        -50        -40        -30
                         M  V  Y  R  R  R  R  S  S  T  G  T
    CATAAATTACACAATTTAAACATGGTTTATCGTCGCCGTCGCCGTTCTTCAACCGGTACC
-20       -10    DraI +1        +10        +20        +30    Asp 718

              AAACGGATCC
              BamH I
```

<p align="center">FIGURE 2</p>

FIGURE 3

FIGURE 4

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | EP-A-0 127 839 (THE TEXAS A & M UNIVERSITY SYSTEM) * The whole document, especially page 11, lines 27-28 * | 1-11 | C 12 N 15/85 C 12 N 15/86 C 12 P 21/00 |
| A,D | JOURNAL OF VIROLOGY, vol. 61, no. 3, March 1987, pages 661-666, American Society for Microbiology; M.E. WILSON et al.: "Location, transcription, and sequence of a baculovirus gene encoding a small arginine-rich polypeptide" * Figure 3 * | 1-11 | |
| A | PROTEIN ENGINEERING, vol. 1, no. 4, August/September 1987, pages 359-366, Eynsham, Oxford, GB; V.C. EMERY et al.: "The development of multiple expression vectors for high level synthesis of eukaryotic proteins: expression of LCMV-N and AcNPV polyhedrin protein by a recombinant baculovirus" * Page 360, last 7 lines; page 361, paragraph 1, figure 1 * | 1-11 | |
| X,P | JOURNAL OF GENERAL VIROLOGY, vol. 71, no. 4, April 1990, pages 971-976, SGM, GB; M.S. HILL-PERKINS et al.: "A baculovirus expression vector derived from the basic protein promoter of Autographa californica nuclear polyhedrosis virus" * The whole article * | 1-11 | |

TECHNICAL FIELDS
SEARCHED (Int. Cl.5)

C 12 N

The present search report has been drawn up for all claims

| Place of search | Date of completion of the search | Examiner |
|---|---|---|
| THE HAGUE | 28-08-1990 | CUPIDO M. |

EPO FORM 1503 03.82 (P0401)